# EUROPEAN PATENT APPLICATION

(11) **EP 4 206 324 A1**
(43) Date of publication of application: **05.07.2023**
(21) Application number: 22159731.3
(22) Date of filing: 02.03.2022
(51) Int. Cl.: C12N 15/10, C12M 1/26, C12M 1/00, C12N 1/06, F04B 43/12

(54) **PURIFICATION OF MACROMOLECULES**

(30) Priority: 04.01.2022 US 202263296224 P
(71) Applicant: Phynexus, Inc., San Jose, CA 95136 (US)
(72) Inventor: GJERDE, Douglas T., Saratoga, 95070 (US); APLAON, Don B., Saratoga, 95070 (US)
(74) Representative: Brann AB

(57) **Abstract**

The present invention relates to the purification of macromolecules, such as plasmids or virus, wherein an automatable method for mixing two or more ingredients in back-and-forth liquid flow mode is used. More specifically, one of the ingredients is a liquid which comprises cells, and one ingredient is either a lysis buffer or a precipitation agent. The invention also includes an automated plasmid purification protocol including automated mixing as an integrated step.

## Description

### Technical field

The present invention relates to the field of macromolecule purification and automatable tools for use therein. More specifically, the present invention relates to an automatable process of purification, such as plasmid purification, wherein cells producing desired macromolecules, such as plasmids, are mixed an agent either for lysis or for the precipitation thereof in an integrated and automated process step. The invention also relates to such an integrated mixing step as such, as well as devices and systems for use in purification of macromolecules.

### Background

One macromolecule which currently is of increasing commercial and scientific interest due to its usefulness as a vector of nucleic acid is the plasmid. Plasmids are small, extrachromosomal DNA molecules within a cell that are physically separated from chromosomal DNA and can replicate independently. Although most plasmids are double-stranded DNA molecules, some consist of single-stranded DNA, or predominantly double-stranded RNA. While chromosomes are large and contain all the essential genetic information for living under normal conditions, plasmids are usually very small and contain only additional genes that may be useful in certain situations or conditions.

Plasmids are commonly used bacterial cloning vectors when large amounts of proteins is desired, and in the more recent field of gene therapy, as it may be arranged e.g. to express a protein that is lacking in mammalian cells. Today, plasmids are highly relevant in vaccine production, for example where genetically engineered plasmids containing a DNA sequence that encodes the antigen(s) against which an immune response is sought is injected into a patient.

However, in plasmid purification and production, lysing and precipitating cells is sometimes difficult due to the danger of shearing delicate genomic DNA as ingredients are mixed, causing the sheared genomic DNA to be recovered together with purified plasmid DNA and rendering the product useless at least for pharmaceutical preparations.

In manually controlled procedures, the mixing of such ingredients may be performed with gentle inversion of the mixing bottle. A dye may be used to show when materials are mixed and no further mixing is needed, which limits the duration of the process to a minimum and as a consequence it will also reduce plasmid shearing.

Currently proposed automated methods include mixing streams, agitating a stream or paddles, and all of these methods have limitations including possible genomic shearing. For example, paddle mixing use mixers that involve high shear velocities at the end of the paddle. Stream mixers rely on turbulent mixing in a tube or chamber which can involve shearing. Thus, previous devices are difficult to integrate into fully automated plasmid purification devices and methods.

EP 0 811 055 (Genzyme Corp) describes as its object to provide an automatable method for isolating high quality plasmids on a large scale to meet the future demand. More specifically, in a first aspect, EP 0 811 055 relates to a method for lysing cells. The method comprises simultaneously flowing a cell suspension and a lysis solution through a static mixer, wherein the cells exit the static mixer lysed. In another aspect, EP 0 811055 relates to a method of simultaneously flowing a cell lysate or other protein-containing solution and precipitating solution through a static mixer, wherein the lysate or protein solution exits the static mixer with its precipitable components precipitated. The two methods may be combined by using static mixers in series. In an embodiment presented as preferred, suitable static mixers contain an internal helical structure which causes two liquids to come in contact with one another in an opposing rotational flow causing the liquids to mix together in a turbulent flow. One stated advantage of EP 0 811 055 is that large volumes of cells can be gently and continuously lysed in-line, greatly simplifying the process of isolating plasmids from large volumes of material without damaging plasmid DNA.

US 5,330,914 (CEMU Bioteknik) relates to a method for automatic purification of extra-chromosomal DNA such as plasmids from a cell suspension, which comprises introducing the cell suspension into a chamber,
introducing a lysing solution into the chamber and mixing it with the contents thereof to lyse the cells therein,
introducing a precipitating solution into the chamber and mixing it with the contents thereof to precipitate chromosomal DNA and, optionally, proteins and cell debris therein,
filtering the contents of the chamber through a filter and feeding the liquid contents to a collecting device,
purifying extra-chromosomal DNA from the contents of the collecting device,
introducing a dissolving solution into the chamber and mixing it with the contents thereof to dissolve the precipitate remaining therein, and
feeding the dissolved precipitate from the chamber to waste.

A mixing chamber is described as provided with agitating means, e.g. vibrating or rotating means, for mixing the cell suspension and the lysing solution.

US 2005/0026177 (Boehringer Ingelheim) relates to a scalable process and device for producing a biomolecule, in particular pharmaceutical grade plasmid DNA. One object of US 2005/0026177 is to provide an automatable and scalable process for isolating a polynucleotide of interest, in particular plasmid DNA, on a manufacturing scale that includes, as a cell disintegration step, an improved alkaline lysis method. US 2005/0026177 describes how several mixing techniques were tested in preliminary experiments, after which it was found that a tube filled with glass beads leads to sufficient mixing and contacting of two solutions consisting of a cell suspension and a lysis solution, respectively. Further, US 2005/0026177 teaches that the described process may b run continuously and fully automated.

WO 2012/134440 (Gjerde et al) relates to methods and devices for sample preparation, such as separating, extracting or purifying nucleic acids, such as DNA and RNA, and more particularly plasmids. The method is described as highly automatable and intended for purifying nucleic acids in a pipette tip column format. However, it is clear from WO 2012/134440 that the term 'automatable' as used therein refers to the purification procedure that begins with the cell pellet, immediately after the lysis step.

Despite the existing methods, there is still a need in the area of plasmid production of methods wherein preferably all steps are automated, at least wherein the important step of mixing cells with chemicals for the lysis thereof is automatable with a small or at least reduced risk of plasmid shearing or other damage. A co-existing need is for the actual hardware that can enable such methods, for example novel mixers which treat delicate plasmids gently.

### Summary of the present invention

The present invention relates to a method as defined in the appended claims. Further details and advantages will follow from the detailed disclosure below, as well as from the dependent claims.

### Brief description of the drawings

Figure 1 shows a schematic and illustrative workflow for automated purification according to the invention including the steps from suspended cells to recovered pellet.
Figure 2 shows how a mixer of the invention, configured for automated back-and-forth-flow mode, may be arranged in more detail.
Figure 3 illustrates in more detail how the liquid flow back and forth may be accomplished as flow up (Figure 3A) and down (Figure 3B), respectively, in a vertically arranged container here constituting the mixing chamber.
Figure 4 illustrates a system including a carousel-type of workstation and configured according to the invention.
Figure 5 illustrates in more detail illustrative steps of a process according to the invention which is performed on a carousel-type of workstation.

### Detailed description of the invention

The present inventors have developed a novel mixer, sometimes herein denoted an Automated Gentle Mixer (AGM), for purification of macromolecules such as plasmids or virus. As will appear from the below, the invention of extracting and preparing cells for plasmid purification is stand alone. However, as the skilled person will appreciate, the inventive method and instrument automation will enable further automation of any other plasmid purification process.

Thus, one advantage of the novel mixer is that it can be fully integrated into an automated purification process. In addition, it is scalable to any desired purification scale. The technology of the invention is based on a back-and-forth flow process.

Another advantage of the invention is that the novel mixer includes a gentle processing of components which are sometimes very delicate, such as plasmids, e.g. supercoiled plasmids, and virus particles. The gently processing is obtained by avoiding the commonly used harsh conditions of turbulent flow and/or mechanical stirrers. Instead, the present invention combines ingredients by folding them together. Thus, "folding" is a term used herein to describe the process of combining ingredients together gently without stirring or otherwise agitating to avoid turbulent flow.

The invention is based on laminar flow automated mixing where flow enters a tube, flows through a tube and expands into a chamber. As the flow expands, the liquid and suspended particulate flow back onto itself, i.e. they fold back to cause mixing. Downward flow is also a laminar process, with liquid and particulate folding back into the mixer. Advantages of the invention is that the process is mechanical but gentle, scalable and integratable.

In a first aspect, the present invention relates to an automatable method for mixing two or more ingredients in a mixing chamber, wherein a first ingredient is a liquid which comprises cells; and a second ingredient is either a lysis buffer or a precipitation buffer. Said mixing is obtained by providing back-and-forth liquid flow within said mixing chamber. Back-and-forth flow mode is obtained be repeating aspiration and dispensing within the mixing chamber any number of times, such as 1-10 times, e.g. 2-5 times.

Back-and-forth flow mode is a well known principle primarily in chromatography, where it is sometimes known as dual flow chromatography, see e.g. US 7,482,169 (Gjerde et al). Thus, the skilled person can easily configure commercially available hardware to perform the claimed mixing using back-and-forth flow mode.

Thus, the present invention is based on the finding that back-and-forth flow mode may be configured in a way that produces a fully satisfactory mixing of liquid components within protocols for the purification of macromolecules, such as plasmids or virus. One major advantage of this flow is that it renders such a protocol fully automatable by integrating the automatic mixing of the invention into any automated prior art process, avoiding the manual and consequently time-consuming careful turning of containers which up to now has been required for delicate macromolecules.

Most conveniently, each ingredient is added to a tube after which they are added to the top or upper part of the mixing chamber, allowing them to expand therein. The mixing chamber, sometimes herein denoted the expansion chamber, may be arranged in any suitable container. One advantage of the invention is that the mixer is easily disposed of. In addition, cleaning thereof is easier than prior art equipment, because the mixer of the invention may be stand alone and not integrated into valves tubing etc.

As the skilled person will appreciate, the volume of the expansion chamber is advantageously large enough to process the entire sample that is mixed.

For large scale applications, column volumes of 1L (Giga scale) or 10L (Tera scale) may be appropriate, with bed volumes of 25mL and 259mL, respectively for each scale.

In an illustrative example, the mixer volume may be about 1 L, while the tube dimensions are about 4-8 mm i.d. tube. As the skilled person will appreciate, smaller and larger tubes would be used as the mixer chamber dimension changes, such as for smaller and larger plasmid preparations. To this end, standard tubing and other equipment is easily selected and adapted as appropriate by the skilled person. For example, the dimensions of tubing and/or mixing chambers are selected to provide the desired degree of laminar liquid flow, for example, a liquid flow which is almost entirely laminar.

Thus, in an advantageous example, the conditions, hardware and settings are configured to provide for substantially laminar flow within the mixing chamber, and optionally the other parts of the set-up used. Thus, the present inventors unexpectedly found that the more gentle conditions of laminar flow mixing are efficient for the mixing of liquids such as cell suspensions, the contents of which may be damaged by prior art protocols utilizing harsher turbulent flow, e.g. as obtained with mechanical stirring means.

In one example, the flow within the mixing chamber will allow for some turbulent flow.

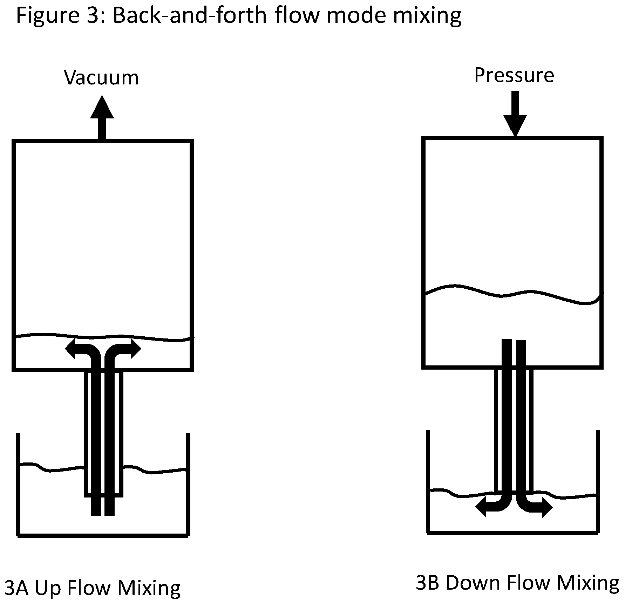

In an advantageous example, as illustrated if Figure 3A and Figure 3B, respectively, vacuum is arranged to provide for aspiration of liquid upwards, while pumping may provide for dispensing liquid downwards. Gently mixing will occur in both directions. Any suitable equipment may be used to this end, such as a peristaltic pump. As the skilled person will appreciate, the way the back-and-forth flow is accomplished may be obtained in many different ways, depending e.g. on the desired degree of mixing as well as the nature of the liquid comprising cells, sometimes herein denoted the 'cell liquid', and is not a limiting feature of this invention.

Back-and-forth flow mode is obtained be repeating aspiration and dispensing within the mixing chamber any number of times, such as 1-10 times, e.g. 2-5 times.

The liquid which comprises cells, i.e. the cell liquid, such as a cell suspension, may have been subjected to one or more steps of pre-treatment and/or conditioning before being added to the mixing chamber of the invention. Such preceding steps may e.g. be a pH-conditioning, centrifugation, decantation of some liquid or any other appropriate measure which is commonly used in the field.

In the present application, the term "lysis buffer" is used in a broad context, and may be any additive that is capable of lysing the cells with which it is combined. Advantageously, the lysis buffer comprises DNase or RNase.

In the present application, the term "precipitation buffer" is used in a broad context, and includes a precipitation agent in any form. Thus, there is no requirement of any pH-buffering effect of the "precipitation buffer" as used herein, it is an ingredient capable of providing precipitation.

The mixing of the invention can be used for any time of lysis and precipitation sample preparation chemistry, including preparation for chaotropic plasmid purification or ion exchange plasmid purification.

If a lysis buffer is mixed with the cell liquid, the cells will advantageously undergo lysis within the mixing chamber. The duration of such a lysis step will be adjusted due to factors well known to the skilled person in this field, such as cell type and lysis agent, liquid flow rate etc. Such cells may be eukaryotic or prokaryotic cells, such as bacterial cells. Advantageous cells are recombinant cells which have been produced to express certain genes or proteins which in their purified form are useful in pharmaceutical applications such as therapy or vaccines. Thus, the lysed cells may release macromolecules, such as virus and/or plasmids. Advantageously, the released macromolecules are plasmids, such as supercoiled plasmid DNA.

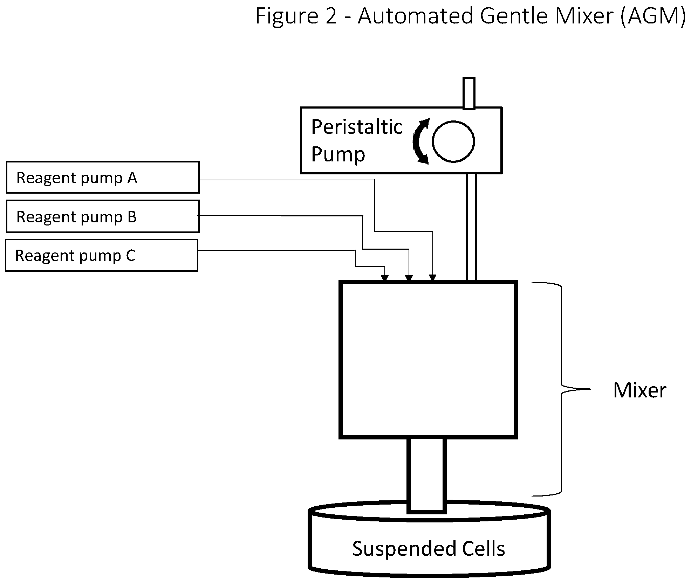

In addition to the cell liquid and lysis buffer and/or precipitation buffer, the present method may also comprise at least one additional ingredient, such as a wash buffer(s). Such additional ingredients may be reagents which are pumped to the mixing chamber separately, as illustrated in Figure 2. The skilled person can conclude what additional ingredients are suitable depending e.g. on the cells as well as on the desired macromolecule, based on common general knowledge.

Further, various ingredients may be added to the mixing chamber after the initiated mixing, i.e. after one or more cycles of back-and-forth flow. For example, after a first period of mixing, a precipitation buffer may be added.

In a second aspect, the present invention relates to an automatable process for purifying macromolecules from a liquid comprising cells, which process comprises mixing the cell liquid at least with either a lysis buffer or a precipitation buffer in back-and-forth liquid flow mode within a mixing chamber to produce a liquid mixture including lysed and/or precipitated cells. Once mixed, the lysed or precipitated cells may be subjected to any commonly used further step for purification, such as a membrane, bead, fibre or column step.

In an illustrative example, the liquid mixture obtained from the mixing chamber is passed across at least one filter to produce a filtrate.

Said filtrate may then be passed across a chromatography column to capture said macromolecules; and the macromolecules may be eluted from the chromatography column.

In an advantageous example, the process of the invention is the herein described method of mixing. Thus, all details discussed above in relation to the first aspect of the invention may be equally applicable to this second aspect.

The filter may be any suitable filter which is automatable and capable of separating macromolecules from other components present in the liquid that exits the mixing chamber, such as a gravity filter. Examples of components separated by the filtering step are cell debris, flocculants and certain larger proteins. Though the process of the invention may include more than one filter without deviating from the scope and spirit of the invention, an advantageous and automatable example comprises a single step of filtration, which is based on the principle of gravity filtration. In order to maximize the yield of macromolecules, the filtering step may include washing of the filter by addition of a suitable wash liquid after passage of the mixed liquid.

The chromatography column may be any suitable column capable of separating macromolecules from other components present in the filtered liquid, such as any still remaining cell debris or flocculants as well as smaller proteins. For example, a chromatography column can be configured to selectively separate desired macromolecules from endotoxins, undesired virus or parts thereof. Equipment for this step is commercially available on the market and may easily be adapted by the skilled person In an advantageous example, the passing of the liquid across a chromatography column includes back-and-forth flow. As discussed above, such flow is well known in the field of chromatography, and the skilled person can easily choose optimal conditions for a devised separation.

A last step of the process may be optional ethanol precipitation or an (ethanol) column concentration. The ethanol also contains high concentration of salt, usually potassium or sodium acetate. Thus, in an illustrative example, a process according to this second aspect of the invention may be followed by a step of concentration to produce a solid precipitate.

As discussed above, one major advantage of the present invention is that it enables automated mixing of cell liquids with other ingredients such as lysis buffers. Thus, in one example of the present process, at least the mixing, and preferably also the filtering and chromatography, are configured to be automated.

In a third aspect, the invention is an automated system comprising a workstation configured for purifying macromolecules produced in cell cultures, which comprises at least a mixing column configured to perform back-and-forth liquid flow; a filter configured to perform gravity filtration; and a liquid chromatography column which is optionally configured to perform for back-and-forth liquid flow.

In an advantageous example of the system of the invention, the workstation is a carousel i.e. a system enabling for continuous circular processing of a cell culture liquid to purify desired macromolecules such as plasmids or virus.

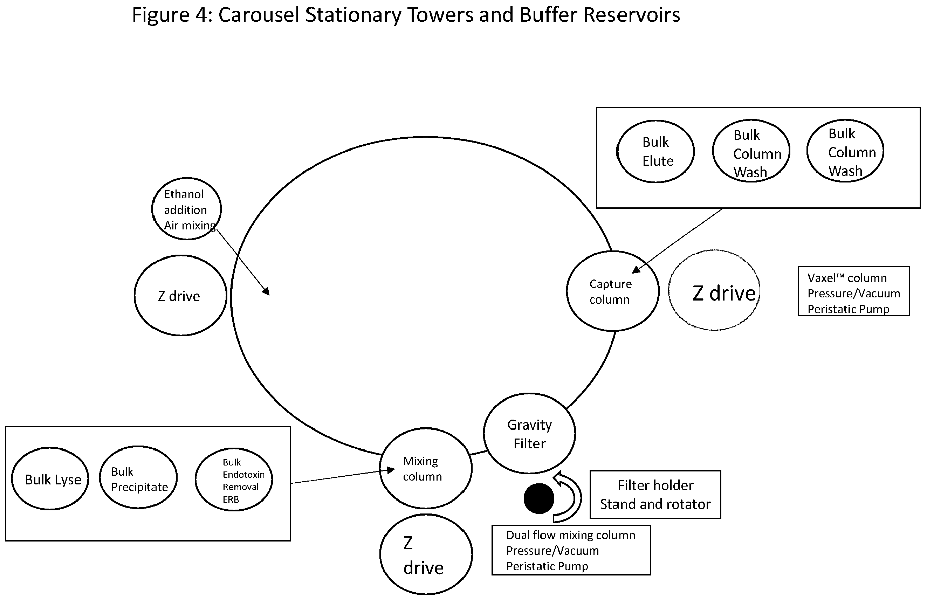

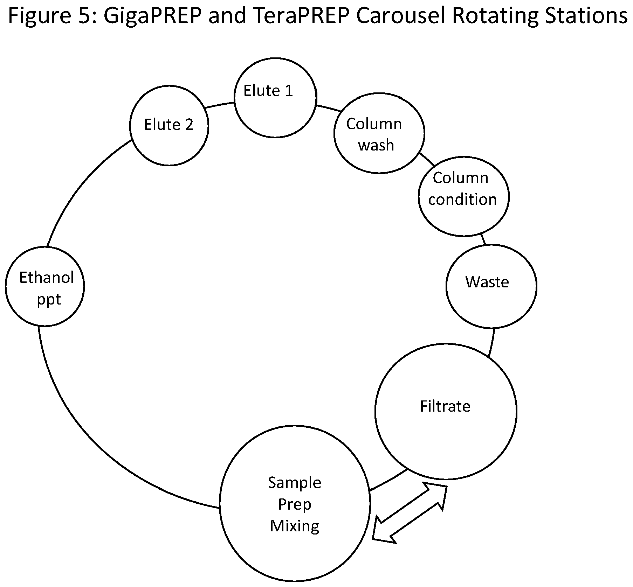

One example of a system including a carousel-type workstation is illustrated in Figures 4 and 5, the detailed description of which will provide further details of this aspect of the invention. As the skilled person will appreciate, details discussed in relation to the first and the second aspect of the invention are equally applicable to this third aspect of the invention.

Thus, a system according to the invention may also comprise containers holding liquids such as eluent and wash liquid. The system may be configured for a purification at a scale of GigaPrep or TeraPrep, which terms are commonly used and well known in this field.

The modules of the system may be Z drive mounted, in accordance to well known principles. Advantageously, the workstation is designed for a specific use, to fully benefit from all advantages while optimizing the speed of processing.

For example, a 2 tier carousel may be used, which is top-level stable and bottom level mobile.

### Detailed description of the drawings

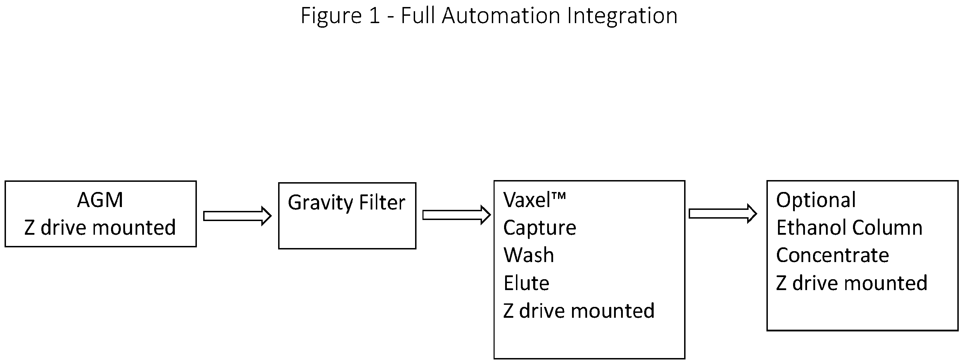

Figure 1 shows a schematic and illustrative workflow for automated purification according to the invention including the steps from suspended cells to recovered pellet. Thus, this is an example of a full automation integrating even the mixing step may be accomplished according to the invention. More specifically, Figure 1 shows an automated gentle mixer (AGM) according to the invention as Z drive mounted; followed by a gravity filter, a Z drive mounted chromatography column, such as a Växel^{™} column (Biotage AB) configure to enable capture of macromolecules as well as washing thereof before elution, and finally the optional step of Z drive mounted concentration, here shown as an ethanol column.

Figure 2 shows how a mixer of the invention, configured for automated back-and-forth-flow mode, may be arranged in more detail. Separate lines have been arranged for separate ingredients, here illustrated as Reagent A, Reagent B and Reagent C, which are all independently provided to the mixing chamber by separate reagent pumps.

Figure 3 illustrates in more detail how the liquid flow back and forth may be accomplished as flow up (Figure 3A) and down (Figure 3B), respectively, in a vertically arranged container here constituting the mixing chamber.

Figure 4 illustrates a system including a carousel-type of workstation and configured according to the invention with GigaPREP and TeraPREP Stationary Towers and containers as buffer reservoirs. Here, the invention is illustrated with a gravity filter and Växel^{™} chromatography columns.

Figure 5 illustrates in more detail illustrative steps of a process according to the invention which is performed on a carousel-type of workstation including process steps of mixing and filtration followed by column chromatography, wash of the column before two subsequent steps of selective elution and an ethanol concentration.

### EXPERIMENTAL

The present examples are provided for illustrative purposes only, and should not be construed to be limiting the invention as defined by the appended claims. All references provided below and elsewhere in the present application are hereby included herein via reference.

### Example 1: Plasmid purification - Fully automated Sample Flow

For this example, the skilled person may use a commercially available workstation adapted with the required hardware elements of the invention.
1. Resuspend 15 g pellet, glass beads, RNase and dye, 87 mL shake. Pour into sample container in the instrument. It is possible that cell pellet resuspension could be automated with dual flow mixing column, but this step requires rigorous mixing. Dual flow mixing column is for gentle mixing and this technology is better suited for gentle lysing and precipitation to prevent genomic DNA shearing.
2. Pump in lysis buffer, 103 mL and dual flow mix.
   Notes:
3. Column head pressure, vacuum and pumping volumes are controlled by peristatic pump with attachment at top of column
4. Pump in precipitation buffer, 123 mL dual flow mix.
5. Pump in 25 mL ERB endotoxin removal buffer, very gentle dual flow mix, one cycle. Do not want bubbles forming. ERB is a surfactant. Do not want to shear.
   Notes:
   Pump to top of dual flow mixing column
   Pumps are peristaltic one for each bulk reagent.
   Consumables are bulk: lysis, precipitation, ERB and possible aqueous filtrate wash

6. Aspirate mixture into dual flow mixing column and rotate carousel (Fig 4 and 5) clockwise to position gravity filter with processing reservoir under the dual flow mixing column. Recover 300 mL filtrate.
   Notes: operations 5 and 6 are performed at the same time
7. Equilibrate 20 mL bed column 60 mL equilibration buffer. Pump 60 mL into top of column and dual flow mix. Dispense to waste. (Filtrate is still under filter with sample. and column is at conditioning position with equilibration reservoir below)
   Notes:
8. Rotate counter clockwise to position filtrate sample under capture column. Capture entire sample with 8 dual flow cycles.
   Notes:
   Column head pressure and vacuum is controlled by peristatic pump with position at top of column
9. Rotate carousel clockwise to wash sample reservoir, pump in 200 mL and dual flow wash.
   Notes:
   Wash may be repeated. Wash may be top down flow.
10. Rotate carousel clockwise to elute reservoir. Pump in 60 mL and dual flow elute. Use 200 mL plastic capture bottle.
   Notes:
   Elute may be top down flow.
11. Capture of the same sample may be repeated using sample. For every capture cycle, we may need an additional wash position and elution position. In this example, we have 3 elution positions.
12. Rotate carousel clockwise and pump in ethanol for precipitation. Insert tube to pump ethanol. Use bubble mixing

## Claims

1. An automatable method for mixing two or more ingredients in a mixing chamber, wherein a first ingredient is a liquid which comprises cells; and a second ingredient is either a lysis buffer or a precipitation buffer; wherein said mixing is obtained by providing back-and-forth liquid flow within said mixing chamber.

2. A method according to claim 1, wherein at least the first ingredient and the second ingredient are added to a tube after which they are added to the top of the mixing chamber and allowed to expand therein.

3. A method according to claim 1 or 2, wherein the provision of back-and-forth liquid flow comprises aspiration of liquid by applying vacuum to the mixing chamber.

4. A method according to any one of claims 1-3, wherein the provision of back-and-forth liquid flow comprises dispensing of liquid by applying a pump action to the mixing chamber.

5. A method according to claim 4, wherein the vacuum and pumping, respectively, are applied sequentially in one or more repeats, such as 2-10 repeats, e.g. 4-6 repeats.

6. A method according to any one of the preceding claims, wherein the liquid which comprises cells is a cell suspension.

7. A method according to any one of the preceding claims, wherein the second ingredient is a lysis buffer which comprises DNase or RNase.

8. A method according to claim 7, wherein the cells of the liquid which comprises cells are lysed within the mixing chamber.

9. A method according to claim 8, wherein said cells release macromolecules, such as virus and/or plasmids, during the lysis thereof.

10. A method according to any one of the claims 1-6, wherein the second ingredient is a precipitation buffer.

11. An automatable process for purifying macromolecules from a liquid which comprises cells, which process comprises mixing said liquid either with a lysis buffer or with a precipitation buffer in back-and-forth liquid flow mode within a mixing chamber to produce a liquid mixture including lysed or precipitated cells; and, optionally, passing said liquid mixture across at least one filter to produce a filtrate.

12. A process according to claim 11, wherein the liquid that comprises cells is mixed with a lysis buffer; the liquid mixture is passed across a filter to produce a filtrate; and said filtrate is passed across a chromatography column to capture or to concentrate said macromolecules; and macromolecules are eluted from the chromatography column.

13. A process according to claim 11 or 12, wherein the mixing is a method according to any one of claims 1-12.

14. A process according to any one of claims 11-13, wherein the filter is a gravity filter.

15. A process according to any one of claims 12-14, wherein the passing of the liquid across a chromatography column includes back-and-forth liquid flow.

16. A process according to any one of claims 11-15, wherein at least the mixing, and preferably also the filtering and capture are configured to be automated process steps.

17. An automated system comprising a workstation configured for purifying macromolecules produced by cells, which comprises at least a mixing column configured to perform back-and-forth liquid flow; a filter configured to perform gravity filtration; and a liquid chromatography column configured to perform for back-and-forth liquid flow.
